# EUROPEAN PATENT SPECIFICATION

(11) **EP 2 743 262 B1**
(45) Date of publication and mention of the grant of the patent: **27.04.2016**
(21) Application number: 12196477.9
(22) Date of filing: 11.12.2012
(51) Int. Cl.: C07D 251/70, C08K 5/3492

(54) **Method for Obtaining a Triazine Compound with at least one Tertiary Amino Group**
Verfahren zum Erhalten einer Triazinverbindung mit mindestens einer tertiären Aminogruppe
Procédé d'obtention d'un composé triazine avec au moins un groupe amino tertiaire

(43) Date of publication of application: 18.06.2014
(73) Proprietor: Borealis Agrolinz Melamine GmbH, 4021 Linz (AT)
(72) Inventor: Dicke, René, 4060 Leonding (AT)
(74) Representative: Maikowski & Ninnemann

(56) References cited:
- WO-A1-94/15257
- NL-A- 9 201 891

## Description

The invention relates to a method for obtaining a triazine compound according to the preamble of claim 1.

### Description

Alkylated triazines such as alkylated melamines are well known and have a broad field of application. For instance, methyl melamines as only one example of alkylated melamines are used as a pharmaceutical compound in cancer therapy, as biocides or insecticides (Calgon 1967, Cassella 1965), as a resin component (Nissan 1995, BASF 1964), as hardener or linking agent (Nissan 1994, Allied Chemistry 1964, Monsanto 1957, Cytec 2003) as an ingredient of stabilizing mixtures for plastics (Nissan 1995, American Cyanamid 1968), in the production of polyimides (Kanebo 1982) as well as in flame retardants, textiles and ion exchange resins.

Due to the broad application spectrum, there is a desire to provide a convenient method for obtaining alkylated triazines such as alkylated melamines with high yields, low impurity content and tailor-made degree of substitution, such as for instance adjusting the ratio of dialkylated N,N-melamine (DMM) and tetraalkylated N,N,N',N'-melamine (TMM).

Different approaches of synthesizing alkylated triazines such as methyl melamine derivatives have been described in the past. Only exemplarily the following methods are mentioned:
- Conversion of melamine with methyl amine salts (IG Farben, Nissan),
- catalytic hydrogenation of methyl etherified melamine formaldehyde resins using suitable catalysts such as Raney nickel (Casella),
- reductive alkylation of melamine by converting melamine with aldehydes / ketones in the presence of a suitable catalyst and hydrogen (Nissan),
- alkylation of melamine with alkyl halogenids using a basic catalyst (Nissan 1995),
- catalytic alkylation of melamine with olefins (Nissan 1994) or
- conversion of cyan guanidine (dicyandiamide) with dimethyl sulphate as methylating agent, methyl amines or methyl cyanamides (American Cyanamid 1953).

Typically, alkylation of triazines occurs at the free amino groups present on the triazine aromatic ring. For instance, melamine as the most well-known triazine compound comprises three primary amino groups, which can in general be alkylated. When using the known methods for alkylating these amino groups when using for instance alkyl halogenids typically each of the primary amino groups is converted at first to its corresponding secondary amino group and only after all of the available amino groups are converted into their corresponding secondary amino groups a further alkylation to their corresponding tertiary amino groups takes place. Thus, it is difficult to provide an alkylated triazine, such as an alkylated melamine, which for instance comprises only one alkylated tertiary amino group and two unsubstituted primary amino groups. The methods so far known are only able, if at all, to provide such specific alkylated triazines in a low yield and only as side products.

It was therefore an object of the present invention to provide a method which allows for a specific synthesis of substituted triazines, in which specifically one of the primary amino groups is at first transaminated for providing a corresponding tertiary amino group before any of the other available primary amino groups are also transaminated.

This object is being solved with the method comprising the features of claim 1.

Accordingly a method for obtaining a triazine compound with at least one amino group thereof being completely substituted, i.e. at least one tertiary amino group, is provided which comprises the steps of
a) providing at least one triazine compound of the general formulae (I) 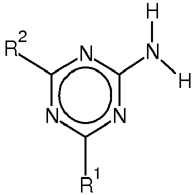 wherein
   - R¹ and R² mean independently from each other Q¹ or a moiety of the formula R³-N-R⁴ or R⁵-N-R⁶ bound with its central nitrogen atom to the triazine ring of the structure of formula (I), whereat
   - Q¹ means H, hydroxyl, a linear or branched C₁-C₃₀-alkyl or a cyclic substituent in form of a C₅-C₂₀-cycloalkyl, a C₅-C₂₀-aryl, a C₁-C₂₀-alkylsubstituted C₅-C₂₀-aryl, wherein in each case one or multiple carbon atoms can be substituted by one or multiple oxygen atoms, sulphur atoms, substituted nitrogen atoms and/or by one or multiple groups of the type - C(O)O-,-OC(O)-,-C(O)- and/or -OC(O)O-, and/or can be functionalized by one or multiple hydroxyl groups and/or mercapto groups;
   - R³, R⁴, R⁵ and R⁶ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O)O-, and/or can be functionalized by one or multiple hydroxyl groups and/or mercapto groups; or an amide of a carboxylic acid or an imide of a cyclic dicarboxylic acid, and
b) reacting said aminotriazine of the general formulae (I) with at least one secondary amine of the general formulae (II)

   (R⁷R⁸)NH

   wherein R⁷ and R⁸ are linear or branched C₁-C₂₀-alkyl, C₅-C₂₀-cycloalkyl, phenyl or C₁-C₂₀-alkylsubstituted C₅-C₂₀-aryl or cyclic amines, or wherein R⁷ and R⁸ together form an alkyl ring, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O) O- and/or can be functionalized by one or multiple hydroxyl groups and/or mercapto groups, and wherein R⁷ and R⁸ can be the same or different;
c) wherein the reaction takes place in the presence of at least one compound of the general formulae (III)

   (R⁹R¹⁰)₂NH₂⁺]ₙXⁿ⁻

   wherein R⁹ and R¹⁰ are linear or branched C₁C₂₀-alkyl, C₅-C₂₀-cycloalkyl, or C₁-C₂₀-alkylsubstituted C₅-C₂₀-aryl or cyclic amines or wherein R⁹ and R¹⁰ together form an alkyl ring, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O) O- and/or can be functionalized by one or multiple hydroxyl groups and/or mercapto groups,wherein R⁹ and R¹⁰ can be the same or different,
   wherein R⁷, R⁸, R⁹ and R¹⁰ can be the same or different,
   wherein Xⁿ⁻ is an anion of an organic or inorganic acid, and
   wherein n ≥1, in particular 1, 2, 3 or 4

The present method allows for the specific modification of a primary amino group of a suitable compound such as a triazine compound, wherein a modification of the N-atom of the primary amino group occurs and thus a tertiary amino group is formed in one step.

This reaction can be described as a transamination reaction wherein a primary amino group of the triazine ring is replaced by the amino group of the secondary amine of formulae (II). In the course of the transamination reaction the N atom of the secondary amine of formulae (II) attacks the C-atom (being δ+) of the triazine ring adjacent to the exocyclic primary amino group. This provides a transition state in which the hydrogen atom of the secondary amino compound of formulae (II) is transferred to the exocyclic primary amino group The simultaneously occurring bond breakage provides the release of ammonia. In other words, the secondary N-atom of formula (II) compound is now bound to the triazine ring and the primary amino group of the triazine is released in form of ammonia.

It is preferred if the R¹ and/or R² are a moiety of the formula R³-N-R⁴ or R⁵-N-R⁶ bound with its central nitrogen atom to the triazine ring of the structure of formula (I), wherein R³, R⁴, R⁵ and R⁶ mean independently from each other H, linear or branched C₁-C₁₀-alkyl (such as methyl, ethyl, propyl, butyl) or C₅-C₁₀-cyclo alkyl (such a cyclopentyl, cyclohexyl), wherein in each case one or multiple carbon atoms can be substituted by one or multiple oxygen atoms, wherein the oxygen atom has at least a distance of (-CH₂-)ₙ with n≥2, such as (-CH₂-CH₂-) from the N-atom, such as morpholine, ethyleneglycol or diethyleneglycol, or can be functionalised with hydroxyl or mercapto groups, such as hydroxyethyl, mercaptoethyl. In the latter case the N-atom is therefore substituted by an ether type moiety.

In a more preferred embodiment of the present method the triazine used in step a) is preferably of the general formulae (Ia) 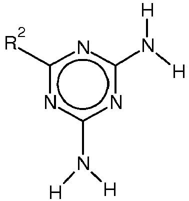 wherein R² has the above meaning. Thus, it may be preferred to use a triazine comprising at least two primary amino groups.

In the case of the compound of formulae (Ia) R¹ is a moiety of the formula R³-N-R⁴ bound with its central nitrogen atom to the triazine ring of the structure of formula (I), wherein R³ and R⁴ mean H.

In a most preferred embodiment of the present method melamine is used as triazine in step a). In this case R¹ and/or R² are a moiety of the formula R³-N-R⁴ or R⁵-N-R⁶ bound with its central nitrogen atom to the triazine ring of the structure of formula (I), wherein R³, R⁴, R⁵ and R⁶ mean H.

In an embodiment of the present method the transamination reaction is carried out in a suitable solvent or solvent free environment. The term "solvent" within the meaning of the present method means thereby a substance which is able to dissolve or dilute a compound without undergoing a chemical reaction between the solvent and the compound to be dissolved. Thus, the present method does not require such a solvent. If the reaction is carried out in a suitable solvent, in particular organic solvent may be selected from a group comprising tetrahydrofuran (THF), dioxane, glycol-dimethylether, ethyleneglycol, diethyleneglycol, alcohols, like ethanol, propanol or butanol, dimethylacetamide, dimethylformamide.

Depending on the type of substituent R⁷, R⁸ of the secondary amine of formulae (II) said secondary amine may be gaseous, liquid or solid. However it was shown that the transamination is best carried by using the at least one secondary amine of the general compound (II) in liquid and/or gaseous form.
When using a secondary amine (II) which may be gaseous at room temperature the liquid of the secondary amine may be obtained by condensing the gaseous amine for instance in the presence of suitable condensation media like dry ice, ice or ice/salt mixtures or such. A second variant is to use such gases as liquefied gases under pressure for direct addition into the reaction vessel.
In a further embodiment of the present method the moieties R⁷, R⁸ of the at least one secondary amine of the general formulae (II) is selected from a group comprising linear or branched C₁-C₁₀-alkyl, C₅-C₁₀-cycloalkyl, or C₁-C₁₀-alkylsubstituted C₅-C₁₀-aryl, wherein in each case one or multiple carbon atoms can be substituted by one or multiple oxygen atoms, sulphur atoms and/or substituted nitrogen atoms and/or can be functionalized by one or multiple hydroxyl groups and/or mercapto groups. Preferred moieties R⁷, R⁸ are methyl, ethyl, n-propyl, isopropyl, n-butyl, s-butyl, t-butyl, isobutyl, pentyl, cyclopentyl, hexyl, cyclohexyl, 2-ethyl-hexyl, octyl, decyl, stearyl, phenyl, toloyl, xyloyl, hydroxyethyl, hydroxypropyl and alike.
In another embodiment the moieties R⁷, R⁸ form together an C₅-C₁₀ alkyl ring, wherein one or multiple carbon atoms can be substituted by one or multiple oxygen atoms, sulphur atoms and/or substituted nitrogen atoms and/or can be functionalized by one or multiple hydroxyl groups and/or mercapto groups. In a preferred embodiment the moieties R⁷, R⁸ are part of morpholine.
In a most preferred embodiment of the present method the secondary amine of formula (II) is selected from group comprising dimethylamine, diethylamine, dipropylamine, dibutylamine, di-*tert-*butylamine, di(hydroxyethyl)amine. In this case R⁷ and R⁸ are the same.
In another preferred embodiment the secondary amine of formula (II) is morpholin or a mixed amine such as N-hydroxyethyl-N-methyl-amine and N-hydroxyethyl-N-ethyl-amine.
The compound of formulae (III) is preferably a salt of the secondary amine of formulae (II); here R⁷, R⁸, R⁹and R¹⁰ are identical.
In such a case the salt may be *in situ* formed by adding a suitable organic or inorganic acid to the reaction mixture of triazine (I) and secondary amine (II), in particular HCl, H₂SO₄, HNO₃ acetic acid, formic acid and others. It is also possible to add the acid at first to the secondary amine (II) and subsequently the triazine is added to this mixture of compounds (II) and (III). However, the salt may also be added separately to the reaction mixture.

It is also possible that a compound of formulae (III) is used, wherein R⁷ R⁸ differ from R⁹, R¹⁰. In such a case the salt (III) is added to the reaction mixture separately. Here the salt (III) may be present in form of an ionic liquid. Typical anions Xⁿ⁻ of such ionic liquids are for instance chloride, nitrate, nitrite, tetrafluorborate, hexafluorphosphate, polyfluoralkansulfonate, bis(trifluormethylsulfonyl)imide, alkylsulfate, alkansulfonate, or acetate.

Thus, it is preferred if the anion Xⁿ⁻ of the compound of formulae (III) is an anion selected from a group comprising halides, in particular chloride, bromide and fluoride, sulfate, phosphate, nitrate, phosphate, nitrite, tetrafluorborate, hexafluorphosphate, polyfluoralkansulfonate, bis(trifluormethylsulfonyl)-, imide, alkylsulfate, alkansulfonate, arylsulfonate, such as toloylsulfonate, formate or acetate.

In a most preferred embodiment of the present method the compound of formulae (III) is selected from group comprising dimethylammonium chloride, diethylammonium chloride, dipropylammonium chloride, dimethylammonium nitrate, diethylammonium nitrate, dipropylammonium nitrate, dimethylammonium sulfate, diethylammonium sulfate, dipropylammonium sulphate, N-hydroxyethyl-N-methyl ammonium chloride, N-hydroxyethyl-N-methyl ammonium nitrate, N-hydroxyethyl-N-methyl ammonium sulphate, N-hydroxyethyl-N-ethyl ammonium chloride, N-hydroxyethyl-N-ethyl ammonium nitrate, N-hydroxyethyl-N-ethyl ammonium sulphate, di-(hydroxyethyl) ammonium chloride, di-(hydroxyethyl) ammonium nitrate, di-(hydroxyethyl) ammonium sulphate, morpholine ammonium chloride, morpholine ammonium nitrate, morpholine ammonium sulphate, ammonium chloride, ammonium bromide, ammoniumsulfate.

In a variant of the present method the ratio of the at least one triazine of general formulae (I) and secondary amine of general formulae (II) is at least 1 : 5, preferably at least 1 : 10, most preferably at least 1: 20. In general a higher amount of the secondary amine increases the reaction velocity.

It is also desirable and preferred, if the amount of the at least one compound of the general formulae (III), i.e. the catalyst, is at least 1 mol%, preferably at least 10 mol%, more preferably at least 50 mol%, most preferably at least 100 mol% in respect to the at least one triazine of the general formulae (I). An increased catalyst amount has a positive influence on the reaction velocity and product composition. However, the compound of formulae (III) is still used in catalytic amounts and thus the compound of formulae (III) does not serve as reaction partner.
The present transamination reaction is preferably carried out at a temperature in a range between 120 and 280°C, preferably between 150 and 230°C, most preferably between 180 and 220°C. In general the conversion rate of the triazine increases with temperature, i.e. the higher the temperature the higher the yield of the transaminated product. This is most probably due to an increased solubility of the triazine used as starting compound in the reaction mixture.
It is also preferred, if the present reaction is carried out for at least 1 h up to 24 hours. The reaction time is preferably selected in dependency on the desired product ratio, in particular on the desired transamination rate. For example, if -in case of using a triazine with at least two primary amino groups as starting compounds- the reaction is stopped after 4 hours or less then a high yield of a triazine having one amino group substituted with two R⁷ moieties is obtained, and thus a triazine with one tertiary amino group is primarily formed. If the reaction time is over 7 h a triazine with two tertiary amino groups is primarily obtained. Therefore, depending on the reaction time and also of course the other reaction conditions as described it is possible to adjust the substitutional pattern of the triazine.
It is also preferred, if the transamination reaction is carried out in an inert gas atmosphere such as in the presence of helium, nitrogen, argon or in the presence of a gas which does not undergo any reaction at the described conditions such as ammonia. This reduces or even avoids side reactions.

It is also possible and preferred if after the reaction is completed the obtained product mixture is at first neutralised before any further work up steps are carried out. Neutralisation can be done by adding any suitable base such as NaOH or KOH. By doing so the amines and ammonia as well as a part of the side products and triazine are removed whereas the desired products are enriched in the solution.
The raw product, preferably obtained after neutralisation, undergoes further work up such as recrystallisation or extraction using as solvents water or alcohols, such as ethanol, butanol, isopropanol or mixtures thereof. The work up can be for instance carried out in such a way that the raw product is heated and dissolved in a solvent and the suspension is hot filtrated. The filtrate is subsequently cooled and the crystallized product is separated and analyzed.

It should also be pointed out that when using melamine as the triazine starting compound - thus a triazine compound with three primary amino groups - then only two tertiary amino groups are obtained. A transamination of the third primary amino group does not occur and thus the present method does not provide a melamine with three tertiary amino groups such as hexamethylmelamine (HMM).

The reaction mechanism of the transamination is now exemplarily shown using melamine as triazine:

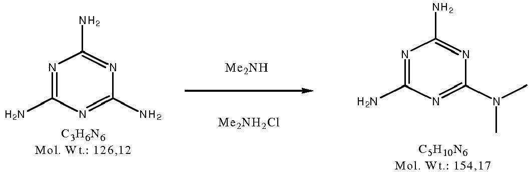

In above reaction scheme one primary amino group of melamine is substituted or undergoes transamination in the presence of dimethylamine and its corresponding chloride salt to N,N-dimethylmelamine (mono-substitution). Depending on the reaction condition such as temperature, reaction time, amount of catalyst, ratio of reactants it is also possible that the reaction proceeds further under formation of N,N,N',N'-tetramethylmelamine (di-substitution).

It is to be understood that above reaction is only one possible example and that the present invention is not restricted to these specific compounds but that rather multiple different triazines and secondary amines may be used as described in detail above.

When applying the reaction conditions as described above at least one compound of the general formulae (IV) 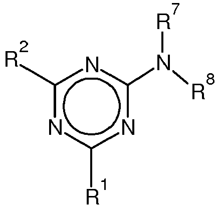 is obtained, wherein the substituents R¹, R² and R⁷, R⁸ have the above meanings.
When using a triazine compound of general formulae (Ia) then preferably a compound of the general formulae (IVa) 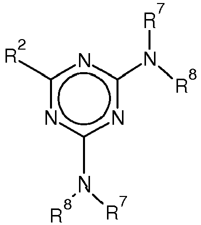 is obtained, wherein the substituents R² and R⁷, R⁸ have the above meanings.
It is in particular preferred that the compound obtained using the present method is selected from a group comprising N,N-dialkylamino triazine and N,N,N',N'-tetraalkylaminotriazine, in particular N,N-dialkylmelamine and N,N,N',N'-tetralkylmelamine. Examples are N,N-dimethylamino triazine and N,N,N',N'-tetramethylamino triazine, in particular N,N-dimethyl melamine and N,N,N',N'-tetramethyl melamine, N,N-diethylamino triazine and N,N,N',N'-tetraethylamino triazine, in particular N,N-diethyl melamine and N,N,N',N'-tetraethyl melamine,
It is in particular preferred that the compound obtained using the present method is selected from a group comprising N-morpholino aminotriazine, N,N'-dimorpholino aminotriazine, in particular N-morpholino melamine, N,N'-dimorpholino melamine, N-hydroxyethyl-N-methyl aminotriazine, N,N,N',N'-di-(hydroxyethyl-methyl) aminotriazine, N-hydroxyethyl-N-ethyl aminotriazine, N,N,N',N'-di-(hydroxyethyl-ethyl) aminotriazine, in particular, N-hydroxyethyl-N-methyl melamine, N,N,N',N'-di-(hydroxyethyl-methyl) melamine, N-hydroxyethyl-N-ethyl melamine, N,N,N',N'-di-(hydroxyethyl-ethyl) melamine. N,N-di-(hydroxyethyl)-amino triazine and N,N,N',N'-tetra-(hydroxyethyl)-amino triazine in particular N,N-di-(hydroxyethyl) melamine and N,N,N',N'-tetra-(hydroxyethyl) melamine.
The compounds obtained by the present method are in particular useful as additives in triazine-formaldehyde resins, such as melamine-formaldehyde-resins, melamine-urea-formaldehyde resins or urea-formaldehyde resins, for improving flexibility of said resins and for reducing brittleness of the resins and for postforming properties of the formed wood based panels.

In the context of the present invention the term "additives" means that the compounds can be added to the resins without undergoing any further reaction with the resin (for instance if the compound does not comprise any free primary amino group), or that the compounds can undergo a reaction with the resin (for instance if the compound still comprises a primary amino group). In the latter case the additive can also be described as a type of comonomer.

Further details of the invention will be explained by the means of the following examples.

### Example 1:

This example is being carried out with a larger amount of melamine (ratio dimethylamine to melamine 10:1).

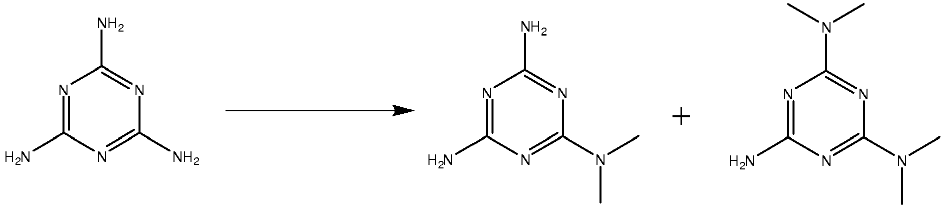

The autoclave beaker is cooled down to at least -5°C using ice / sodium chloride. Dimethylamine (540 g; 12 mol) is added to the beaker, which is previously obtained by condensing the gaseous dimethylamine in a flask cooled with dry ice / isopropanol. Subsequently, dimethyl ammonium chloride (9.8 g; 0.12 mol) and melamine (151.3 g; 1.2 mol) is added in this order to the beaker. The autoclave is closed and the reaction mixture is heated for 8 h at 220°C with strong stirring. After completion of the reaction it is carefully vented and the exhaust gas is fed into water. The resulting material is quenched with aqueous 2N sodium hydroxide solution, filtered, washed with water and dried under reduced pressure.
Amount of raw product obtained: 173.6 g.
HPLC result: Melamine: 13.2 m-%; methyl melamine: m-3.6%; N,N'-dimethyl melamine: 2.2 m-%; N,N-dimethyl melamine: 33.9 m-%; N,N,N',N'-tetramethyl melamine: 47.0 m-%, N,N,N',N'-tetramethyl melamine (TMM) is the main product after 8 h at the selected reaction conditions. Two further tests (see Examples 2 and 3) with a different reaction time were conducted in order to increase the yield of N,N-dimethyl melamine (DMM).

### Example 2:

The same reaction conditions were employed as described for Example 1 with the difference that the reaction mixture is stirred at 220°C for 6 h.
Raw product: 165.6 g.
HPLC: Melamine: 31.4 m-%; methyl melamine: 2.9 m-%; N,N'-dimethyl melamine: 1.3 m-%; N,N-dimethyl melamine: 28.8 m-%; N,N,N',N'-tetramethyl melamine: 35.6 m-%;

The decrease of reaction time in respect to Example 1 provides a lower conversion. However, N,N,N',N'-tetratmethyl melamine is also the main product, what can point to a delayed reaction of the melamine due to bad solubility of the melamine.

### Example 3:

The same reaction conditions as described for Example 1 were employed with the difference that the reaction mixture was stirred at 220°C for 10 h.
Raw product: 199.9 g;
HPLC: Melamine: 2.3 m-%; methyl melamine: 3.0 m-%; N,N'-dimethyl melamine: 1.9 m-%; N,N-dimethyl melamine: 38.7 m-%; N,N,N',N'-tetramethyl melamine: 54.0 m-%

### Example 4:

The autoclave beaker is cooled down to at least -5°C with ice / sodium chloride. Dimethyl amine (540 g; 12 mol) is added, which has been previously obtained by condensing the gaseous dimethyl amine in a flask cooled by dry ice / isopropanol. Subsequently, dimethyl ammonium chloride (48.6 g; 0.6 mol) and melamine (151.3 g; 1.2 mol) is added in this order to the beaker. The autoclave is closed and the reaction mixture is heated for 4 h at 220°C with stirring. After completion of the reaction, it is carefully ventilated and the exhaust gas is fed into water. The resulting material is quenched with aqueous 2N sodium hydroxide solution, filtered, washed with water and dried under reduced pressure.
Raw product: 270.1 g;
HPLC: Melamine: 0.1 m-%; methyl melamine: 1.1 m-%; N,N-dimethyl melamine: 73 m-%; N,N,N',N'-tetramethyl melamine: 24.3 m-%

The increase of the amount of catalyst has a positive effect on DMM yield and the impurity profile. These reaction conditions provide the best yield on DMM. The raw product contains furthermore only a few impurities. A certain amount of melamine and/or TMM is unavoidable, since it is a subsequent reaction. By amending the reaction time the optimal time for interrupting the reaction has to be determined.

A further purification of the raw product was done by recrystallization in n-butanol. The raw product was suspended in 5 times access of n-butanol and heated up to reflux temperature. The hot mixture was filtrated, and the 2 products (filter cake product and crystallized product from n-butanol were dried.
Analysis of filter cake product by HPLC: N,N-dimethyl melamine: 92 m-%; N,N,N',N'-tetramethyl melamine: 5 m-%
Analysis of crystallized product by HPLC: N,N-dimethyl melamine: 16 m-%; N,N,N',N'-tetramethyl melamine: 83 m-%

### Example 5:

The autoclave beaker is cooled down to at least -5°C with ice / sodium chloride. Dimethyl amine (540 g; 12 mol) is added, which has been previously obtained by condensing the gaseous dimethyl amine in a flask cooled by dry ice / isopropanol. Subsequently, dimethyl ammonium chloride (97.2 g; 1.2 mol) and melamine (151.3 g; 1.2 mol) is added in this order to the beaker. The autoclave is closed and the reaction mixture is heated for 8 h at 220°C with stirring. After completion of the reaction, it is carefully ventilated and the exhaust gas is fed into water.

The resulting material is quenched with aqueous 2N sodium hydroxide solution, filtered, washed with water and dried under reduced pressure.
Raw product: 256.5 g;
HPLC: Melamine: 0.1 m-%; methyl melamine: 0.1 m-%; N,N-dimethyl melamine: 0.4 m-%; N,N,N',N'-tetramethyl melamine: 98.2 m-%

### Example 6:

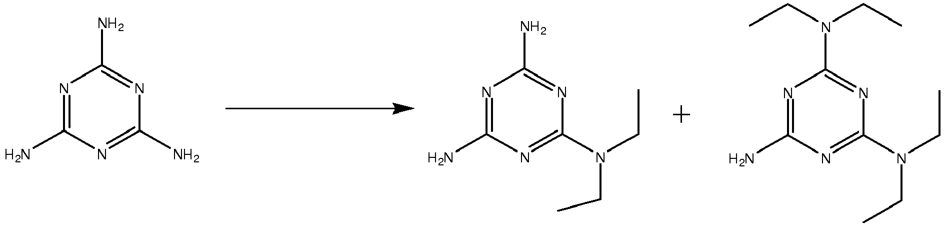

The autoclave beaker is cooled down to at least 5°C with ice. Diethyl amine (365 g; 5 mol) is added. Subsequently, ammonium chloride (27 g; 0.5 mol) and melamine (63 g; 0.5 mol) is added in this order to the beaker. The suspension is again stirred, the autoclave is closed and the reaction mixture is heated for 24 h at 200°C with stirring. After completion of the reaction, it is carefully ventilated and the exhaust gas is fed into water. The unreacted diethylamine is now distilled and the residue is quenched with aqueous 2N sodium hydroxide solution, filtered, washed with water and dried under reduced pressure.
Raw product: 94 g;
HPLC: Melamine: 8.9 m-%; ethyl melamine: 0.9 m-%; N,N-diethyl melamine: 62.9 m-%; N,N,N',N'-tetramethyl melamine: 10.5 m-%

### Example 7:

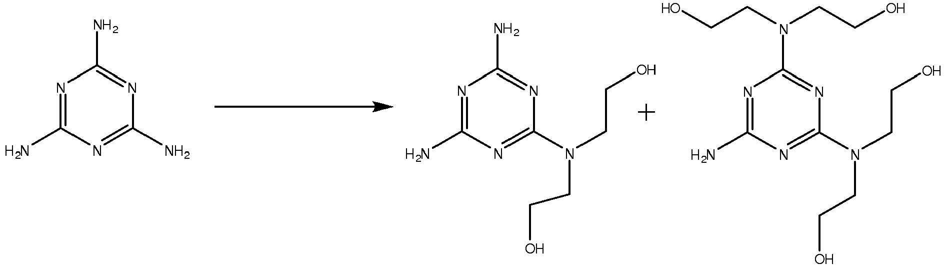

In an autoclave beaker, bis-(hydroxyethyl) amine (52.5 g; 0.5 mol) is added. Subsequently, bis-(hydroxyethyl) ammonium sulphate (1.54 g; 0.005 mol), formed by mixing of sulphuric acid and bis-(hydroxyethyl) amine, and melamine (6.3 g; 0.05 mol) is added to the beaker. The autoclave is closed and the reaction mixture is heated for 4 h at 220°C with stirring. After completion of the reaction, it is carefully ventilated and the exhaust gas is fed into water. The unreacted amine is now distilled under reduced pressure and the residue is quenched with aqueous 2N sodium hydroxide solution, filtered, washed with water and dried under reduced pressure.
Raw product: 12.4g;
HPLC: Melamine: 19 m-%; N,N- bis-(hydroxyethyl) melamine: 61 m-%; N,N,N',N'-tetra-(hydroxyethyl) melamine: 17 m-%

### Example 8:

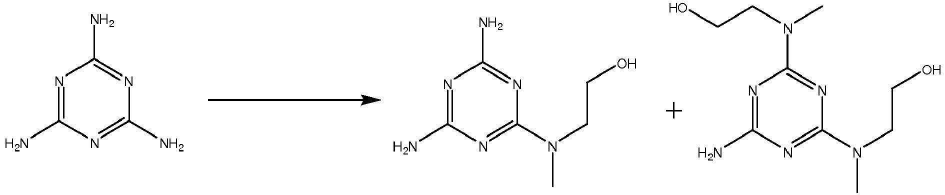

In an autoclave beaker, N-(hydroxyethyl)-N-methyl amine (37.6 g; 0.5 mol) is added. Subsequently, N-(hydroxyethyl)-N-methyl ammonium sulphate (1.24 g; 0.005 mol), formed by mixing of sulphuric acid and N-(hydroxyethyl)-N-methyl amine, and melamine (6.3 g; 0.05 mol) is added to the beaker. The autoclave is closed and the reaction mixture is heated for 24 h at 220°C with stirring. After completion of the reaction, it is carefully ventilated and the exhaust gas is fed into water. The unreacted amine is now distilled under reduced pressure and the residue is quenched with aqueous 2N sodium hydroxide solution, filtered, washed with water and dried under reduced pressure.
Raw product: 11.1 g;
HPLC: Melamine: 2.9 m-%; N,N- (hydroxyethyl)-methyl melamine: 53.7 m-%; N,N,N',N'-bis-(hydroxyethyl)-dimethyl melamine: 34 m-%

### Example 9:

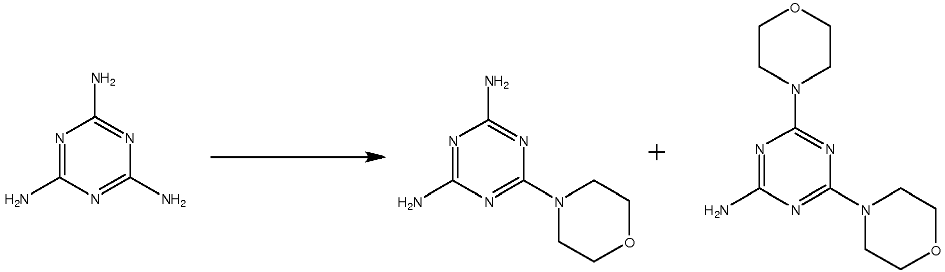

In a autoclave beaker, morpholine (43.6 g; 0.5 mol) is added. Subsequently, morpholine hydrochloride (0.62 g; 0.005 mol) and melamine (6.3 g; 0.05 mol) is added to the beaker. The autoclave is closed and the reaction mixture is heated for 24 h at 200°C with stirring. After completion of the reaction, it is carefully ventilated and the exhaust gas is fed into water. The unreacted morpholine is now distilled under reduced pressure and the residue is quenched with aqueous 2N sodium hydroxide solution, filtered, washed with water and dried under reduced pressure with water.
Raw product: 12.8g;
HPLC: Melamine: 6.7 m-%; Mono-morpholino melamine: 61.5 m-%; Di-morpholino melamine: 28.6 m-%

### Example 10:

In an autoclave beaker dimethylamine (194 g; 4.3 mol) is dissolved in 300 g ethyleneglycol. Subsequently, dimethyl ammonium chloride (34.8 g; 0.43 mol) and melamine (54.2 g; 0.43 mol) is added in this order to the beaker. The autoclave is closed and the reaction mixture is heated for 24 h at 200°C with strong stirring. Now, the reaction is cooled down and after crystallization the product is filtered, washed intensively with water and dried under reduced pressure.
Amount of raw product obtained: 76.4 g.
HPLC result: Melamine: 2.9 m-%; N,N-dimethyl melamine: 53.7 m-%; N,N,N',N'-tetramethyl melamine: 34.0 m-%,

## Claims

1. Method for obtaining a triazine compound with at least one tertiary amino group Comprising the steps of
a) providing at least one triazine compound of the general formulae (I) 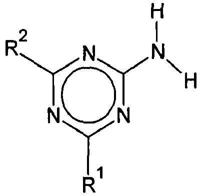 wherein
- R¹ and R² mean independently from each other Q¹ or a moiety of the formula R³-N-R⁴ or R⁵-N-R⁶ bound with its central nitrogen atom to the triazine ring of the structure of formula (I), whereat
- Q¹ means H, hydroxyl, a linear or branched C₁-C₃₀-alkyl or a cyclic substituent in form of a C₅-C₂₀-cycloalkyl, a C₅-C₂₀-aryl, a C₁-C₂₀-alkylsubstituted C₅-C₂₀-aryl, wherein in each case one or multiple carbon atoms can be substituted by one or multiple oxygen atoms, sulphur atoms, substituted nitrogen atoms and/or by one or multiple groups of the type -C(O)O-,-OC(O)-,-C(O)- and/or-OC(O)O-, and/or can be functionalized by one or multiple hydroxyl groups and/or mercapto groups,
- R³, R⁴, R⁵ and R⁶ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O)O-, and/or can be functionalized by one or multiple hydroxyl groups and/or mercapto groups; or an amide of a carboxylic acid or an imide of a cyclic dicarboxylic acid, and
b) reacting said aminotriazine of the general formulae (I) with at least one secondary amine of the general formulae (II)
(R⁷R⁸)NH
wherein R⁷ and R⁸ are linear or branched C₁-C₂₀-alkyl, C₅-C₂₀-cycloalkyl, phenyl or C₁-C₂₀-alkylsubstituted C₅-C₂₀-aryl or cyclic amines, or wherein R⁷ and R⁸ together form an alkyl ring, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or - OC(O) O- and/or can be functionalized by one or multiple hydroxyl groups and/or mercapto groups, and wherein R⁷ and R⁸ can be the same or different;
c) wherein the reaction takes place in the presence of at least one compound of the general formulae (III)
(R⁹R¹⁰)₂NH₂⁺]ₙXⁿ⁻
wherein R⁹ and R¹⁰ are linear or branched C₁-C₂₀-alkyl, C₅-C₂₀-cycloalkyl, or C₁-C₂₀-alkylsubstituted C₅-C₂₀-aryl or cyclic amines or wherein R⁹ and R¹⁰ together form an alkyl ring, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O) O-and/or can be functionalized by one or multiple hydroxyl groups and/or mercapto groups,wherein R⁹ and R¹⁰ can be the same or different,
wherein R⁷, R⁸,R⁹ and R¹⁰ can be the same or different
wherein Xⁿ⁻ is an anion of an organic or inorganic acid, and
wherein n ≥1, in particular 1, 2, 3 or 4

2. Method according to claim 1, **characterized in that** the at least one secondary amine of the general compound (II) is used as a liquid and/or a gas and/or liquefied gas.

3. Method according to one of the preceding claims, **characterized in that** R⁷, R⁸ of the at least one secondary amine of the general formulae (II) is selected from a group comprising linear or branched C₁-C₁₀-alkyl, C₅-C₁₀-cycloalkyl, or C₁-C₁₀-alkylsubstituted C₅-C₁₀-aryl or wherein R⁷, R⁸ form together an C₆-C₁₀ alkyl ring, wherein in each case one or multiple carbon atoms can be substituted by one or multiple oxygen atoms, sulphur atoms and/or substituted nitrogen atoms and/or can be functionalized by one or multiple hydroxyl groups and/or mercapto groups.

4. Method according to claim 3, **characterized in that** R⁷, R⁸ are selected from a group comprising methyl, ethyl, n-propyl, isopropyl, n-butyl, s-butyl, t-butyl, isobutyl, pentyl, cyclopentyl, hexyl, cyclohexyl, 2-ethyl-hexyl, octyl, decyl, stearyl, toloyl, xyloyl, hydroxyethyl, hydroxypropyl or wherein R⁷, R⁸ are part of morpholine.

5. Method according to one of the preceding claims, **characterized in that** that Xⁿ⁻ of the compound of formulae (III) is an anion selected from a group comprising halogenids, in particular chloride, bromide and fluoride, sulfate, phosphate, nitrate, nitrite, tetrafluorborate, hexafluorphosphate, polyfluoralkansulfonate, bis(trifluormethylsulfonyl)imide, alkylsulfate, alkansulfonate, arylsulfonate, acetate.

6. Method according to one of the preceding claims **characterized in that** the ratio of the at least one triazine of general formulae (I) and secondary amine of general formulae (II) is at least 1:5, preferably at least 1:10, most preferably at least 1:20.

7. Method according to one of the preceding claims, **characterized in that** the amount of the at least one compound of the general formulae (III) is at least 1 mol%, preferably at least 10 mol%, more preferably at least 50 mol%, most preferably at least 100 mol% in respect to the at least one triazine of the general formulae (I).

8. Method according to one of the preceding claims, **characterized in that** the reaction is carried out at a temperature in a range between 120 and 280°C, preferably between 150 and 230°C, most preferably between 180 and 220°C.

9. Method according to one of the preceding claims, **characterized in that** the reaction is carried out for at least 1 h up to 24 hours.

10. Method according to one of the preceding claims, **characterized in that** the triazine used in step a) is of the general formulae (Ia) 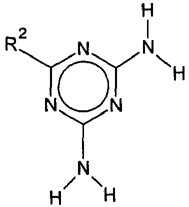 wherein R² has the above meaning.

11. Method according to one of the preceding claims, **characterized in that** melamine is used as triazine in step a).

12. Method according to one of the preceding claims **characterized in that** the compound obtained is of the general formulae (IV) 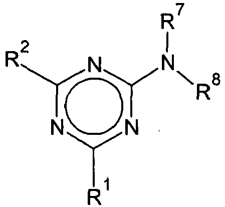 wherein the substituents R¹, R² and R⁷, R⁸ have the above meanings.

13. Method according to one of the preceding claims, **characterized in that** the compound obtained is of the general formulae (IVa) 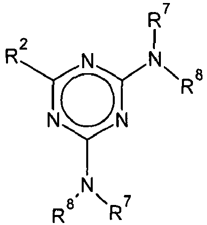 wherein the substituents R² and R⁷, R⁸ have the above meanings.

14. Method according to one of the preceding claims, **characterized in that** the compound obtained is selected from a group comprising N,N-dialkylamino triazine and N,N,N',N'-tetraalkylamino triazine, in particular N,N-dialkylmelamine and N,N,N',N'-tetralkylmelamine, N,N-dimethylamino triazine and N,N,N',N'-tetramethylamino triazine, in particular N,N-dimethyl melamine and N,N,N',N'-tetramethyl melamine, N,N-diethylamino triazine and N,N,N',N'-tetraethylamino triazine, in particular N,N-diethyl melamine and N,N,N',N'-tetraethyl melamine, N-morpholino aminotriazine, N,N'-dimorpholino aminotriazine, in particular N-morpholino melamine, N,N'-dimorpholino melamine, N,N-di-(hydroxyethyl)-amino triazine and N,N,N',N'-tetra-(hydroxyethyl)-amino triazine in particular N,N-di-(hydroxyethyl) melamine and N,N,N',N'-tetra-(hydroxyethyl) melamine, N-hydroxyethyl-N-methyl aminotriazine, N,N,N',N'-di-(hydroxyethyl-methyl) aminotriazine, N-hydroxyethyl-N-ethyl aminotriazine, N,N,N',N'-di-(hydroxyethyl-ethyl) aminotriazine, in particular N-morpholino melamine, N,N'-dimorpholino melamine, N-hydroxyethyl-N-methyl melamine, N,N,N',N'-di-(hydroxyethyl-methyl) melamine, N-hydroxyethyl-N-ethyl melamine, N,N,N',N'-di-(hydroxyethyl-ethyl) melamine.

## Patentansprüche

1. Verfahren zur Herstellung einer Triazin-Verbindung mit mindestens einer tertiären Aminogruppe umfassend die Schritte
a) Bereitstellen von mindestens einer Triazin-Verbindung der allgemeinen Formel (I) 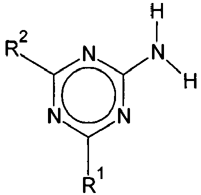 wobei
- R¹ und R² unabhängig voneinander Q¹ oder eine Gruppe der Formel R³-N-R⁴ oder R⁵-N-R⁶, die mit ihrem zentralen Stickstoffatom an den Triazinring der Struktur der Formel (I) gebunden ist, bedeuten, wobei
- Q¹ bedeutet H, Hydroxyl, ein lineares oder verzweigtes C₁-C₃₀-Alkyl oder einen zyklischen Substituenten in Form eines C₅-C₂₀-Cycloalkyl, eines C₅-C₂₀-Aryl, eines C₁-C₂₀-alkylsubstituierten C₅-C₂₀-Aryl, wobei jeweils ein oder mehrere Kohlenstoffatome durch ein oder mehrere Sauerstoffatome, Schwefelatome, substituierte Stickstoffatome und/oder durch ein oder mehrere Gruppen des Typs -C(O)O-, -OC(O)-,-C(O)- und/oder -OC(O)O- substituiert sein können, und/oder mit ein oder mehreren Hydroxylgruppen und/oder Mercaptogruppen funktionalisiert sein können,
- R³, R⁴, R⁵ und R⁶ unabhängig voneinander H, lineares oder verzweigtes C₁-C₂₀-Alkyl, C₅-C₂₀-Cycloalkyl, C₅-C₂₀-Aryl, C₁-C₂₀-alkylsubstituiertes C₅-C₂₀-Aryl bedeuten, wobei jeweils ein oder mehrere Kohlenstoffatome durch ein mehrere Sauerstoffatome, Schwefelatome und/oder substituierte Stickstoffatome und/oder durch ein oder mehrere Gruppen des Typs -C(O)O-, -OC(O)-, -C(O)- und/oder -OC(O)O- substituiert sein können, und/oder mit ein oder mehreren Hydroxylgruppen und/oder Mercaptogruppen, oder einem Amid einer Carboxylsäure oder einem Imid einer zyklischen Dicarboxylsäure funktionalisiert sein können, und
b) Reagieren des Aminotriazins der allgemeinen Formel (I) mit mindestens einem sekundären Amine der allgemeinen Formel (II)
(R⁷R⁸)NH
wobei R⁷ und R⁸ ein lineares oder verzweigtes C₁-C₂₀-Alkyl, C₅-C₂₀-Cycloalkyl, Phenyl oder C₁-C₂₀-alkylsubstituiertes C₅-C₂₀-Aryl oder cyclische Amine sind, oder wobei R⁷ und R⁸ zusammen einen Alkylring ausbilden, wobei jeweils ein oder mehrere Kohlenstoffatome durch ein oder mehrere Sauerstoffatome, Schwefelatome und/oder substituierte Stickstoffatome und/oder durch ein oder mehrere Gruppen des Typs - C(O)O-, -OC(O)-, -C(O)- und/oder -OC(O) O- substituiert sein können und/oder mit ein oder mehreren Hydroxylgruppen und/oder Mercaptogruppen funktionalisiert sein können, und wobei R⁷ und R⁸ gleich oder unterschiedlich sein können;
c) wobei die Reaktion in Gegenwart von mindestens einer Verbindung der allgemeinen Formel (III)
(R⁹R¹⁰)₂NH2⁺]ₙXⁿ⁻
stattfindet, wobei R⁹ und R¹⁰ linear oder verzweigtes C₁-C₂₀-Alkyl, C₅-C₂₀-Cycloalkyl, oder C₁-C₂₀-alkylsubstituiertes C₅-C₂₀-Aryl oder zyklische Amine sind oder wobei R⁹ und R¹⁰ zusammen einen Alkylring ausbilden, wobei jeweils ein oder mehrere Kohlenstoffatome durch ein oder mehrere Sauerstoffatome, Schwefelatome und/oder substituierte Stickstoffatome und/oder durch ein oder mehrere Gruppen des Typs -C(O)O-, -OC(O)-, -C(O)- und/oder -OC(O)O- substituiert sein können und/oder mit ein oder mehreren Hydroxylgruppen und/oder Mercaptogruppen funktionalisiert sein können, wobei R⁹ und R¹⁰ gleich oder unterschiedlich sein können,
wobei R⁷, R⁸, R⁹ und R¹⁰ gleich oder unterschiedlich sein können,
wobei Xⁿ⁻ ein Anion einer organischen oder anorganischen Säure ist, und
wobei n ≥1, insbesondere 1, 2, 3 oder 4 ist.

2. Verfahren gemäß Anspruch 1, **dadurch gekennzeichnet, dass** das mindestens eine sekundäre Amine der allgemeinen Verbindung (II) als eine Flüssigkeit und/oder als ein Gas und/oder ein verflüssigtes Gas verwendet wird.

3. Verfahren gemäß einem der vorhergehenden Ansprüche, **dadurch gekennzeichnet, dass** R⁷, R⁸ des mindestens einen sekundären Amins der allgemeinen Formel (II) ausgewählt ist aus einer Gruppe umfassend lineares oder verzweigtes C₁-C₁₀-Alkyl, C₅-C₁₀-Cycloalkyl, oder C₁-C₁₀-alkylsubstituiertes C₅-C₁₀-Aryl oder wobei R⁷, R⁸ zusammen einen C₆-C₁₀ Alkylring ausbilden, wobei jeweils ein oder mehrere Kohlenstoffatome durch ein oder mehrere Sauerstoffatome, Schwefelatome und/oder substituierte Stickstoffatome substituiert sein können und/oder mit ein oder mehreren Hydroxylgruppen und/oder Mercaptogruppen funktionalisiert sein können.

4. Verfahren gemäß Anspruch 3, **dadurch gekennzeichnet, dass** R⁷, R⁸ ausgewählt sind aus einer Gruppe umfassend Methyl, Ethyl, n-Propyl, Isopropyl, n-Butyl, s-Butyl, t-Butyl, Isobutyl, Pentyl, Cyclopentyl, Hexyl, Cyclohexyl, 2-Ethyl-Hexyl, Octyl, Decyl, Stearyl, Toloyl, Xyloyl, Hydroxyethyl, Hydroxypropyl oder wobei R⁷, R⁸ Teil von Morpholin sind.

5. Verfahren gemäß einem der vorhergehenden Ansprüche, **dadurch gekennzeichnet, dass** Xⁿ⁻ der Verbindung der Formel (III) ein Anion ausgewählt aus einer Gruppe umfassend Halogenide, insbesondere Chlorid, Bromid und Fluorid, Sulfat, Phosphat, Nitrat, Nitrit, Tetrafluorborat, Hexafluorphosphat, Polyfluoralkansulfonat, Bis(tri-Fluormethylsulfonyl)imide, Alkylsulfat, Alkansulfonat, Arylsulfonat, Acetat ist.

6. Verfahren gemäß einem der vorhergehenden Ansprüche, **dadurch gekennzeichnet, dass** das Verhältnis des mindestens einen Triazins der allgemeinen Formel (I) und des sekundären Amins der allgemeinen Formel (II) mindestens 1:5, bevorzugt mindestens 1:10, noch bevorzugter mindestens 1:20 ist.

7. Verfahren gemäß einem der vorhergehenden Ansprüche, **dadurch gekennzeichnet, dass** die Menge der mindestens einen Verbindung der allgemeinen Formel (III) mindestens 1 mol%, bevorzugt mindestens 10 mol%, noch bevorzugter mindestens 50 mol%, am meisten bevorzugt mindestens 100 mol% in Bezug auf das mindestens eine Triazin der allgemeinen Formel (I) ist.

8. Verfahren gemäß einem der vorhergehenden Ansprüche, **dadurch gekennzeichnet, dass** die Reaktion bei einer Temperatur in einem Bereich zwischen 120 und 280°C bevorzugt zwischen 150 und 230°C, am meisten bevorzugt zwischen 180 und 220°C ausgeführt wird.

9. Verfahren gemäß einem der vorhergehenden Ansprüche, **dadurch gekennzeichnet, dass** die Reaktion für mindestens 1 Stunde bis zu 24 Stunden durchgeführt wird.

10. Verfahren gemäß einem der vorhergehenden Ansprüche, **dadurch gekennzeichnet, dass** das in Schritt a) verwendete Triazin die allgemeine Formel (la) 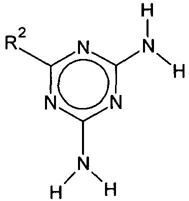 hat, wobei R² die obige Bedeutung aufweist.

11. Verfahren gemäß einem der vorhergehenden Ansprüche, **dadurch gekennzeichnet, dass** in Schritt a) Melamin als Triazin verwendet wird.

12. Verfahren gemäß einem der vorhergehenden Ansprüche, **dadurch gekennzeichnet, dass** die erhaltene Verbindung die allgemeine Formel (IV) 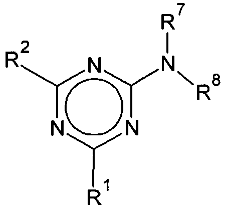 aufweist, wobei die Substituenten R¹, R² und R⁷, R⁸ die obige Bedeutung aufweisen.

13. Verfahren gemäß einem der vorhergehenden Ansprüche, **dadurch gekennzeichnet, dass** die erhaltene Verbindung die allgemeine Formel (IVa) 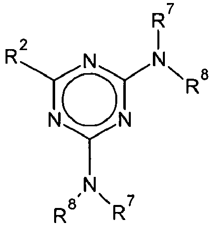 aufweist, wobei die Substituenten R² und R⁷, R⁸ die obige Bedeutung haben.

14. Verfahren gemäß einem der vorhergehenden Ansprüche, **dadurch gekennzeichnet, dass** die erhaltene Verbindung ausgewählt ist aus einer Gruppe umfassend N,N-Dialkylaminotriazine und N,N,N',N'-Tetraalkylaminotriazine, insbesondere N,N-Dialkylmelamin und N,N,N',N'-Tetralkylmelamin, N,N-Dimethylaminotriazine und N,N,N',N'-Tetramethylaminotriazine, insbesondere N,N-Dimethylmelamin und N,N,N',N'-Tetramethylmelamin, N,N-Diethylaminotriazine und N,N,N',N'-Tetraethylaminotriazine, insbesondere N,N-Diethylmelamin und N,N,N',N'-Tetraethylmelamin, N-Morpholinaminotriazine, N,N'-Dimorpholinaminotriazin, insbesondere N-Morpholinmelamin, N,N'-Dimorpholinmelamin, N,N-Di-(Hydroxyethyl)-aminotriazine und N,N,N',N'-Tetra-(Hydroxyethyl)-aminotriazine insbesondere N,N-Di-(Hydroxyethyl)melamin und N,N,N',N'-Tetra-(Hydroxyethyl)melamin, N-Hydroxyethyl-N-Methylaminotriazine, N,N,N',N'-Di-(Hydroxyethyl-methyl)aminotriazine, N-Hydroxyethyl-N-Ethylaminotriazine, N,N,N',N'-Di-(Hydroxyethyl-Ethyl)aminotriazine, insbesondere N-Morpholinmelamin, N,N'-Dimorpholinmelamin, N-Hydroxyethyl-N-Methylmelamin, N,N,N',N'-Di-(Hydroxyethyl-Methyl)melamin, N-Hydroxyethyl-N-Ethylmelamin, N,N,N',N'-Di-(Hydroxyethyl-Ethyl)melamin.

## Revendications

1. Procédé d'obtention d'un composé triazine avec au moins un groupe amino tertiaire, comprenant les étapes consistant à
a) fournir au moins un composé triazine de formule générale (I) 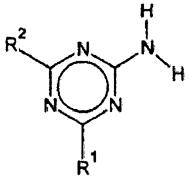 dans laquelle
- R¹ et R² signifient, indépendamment l'un de l'autre, Q¹ ou un fragment de formule R³-N-R⁴ ou R⁵-N-R⁶ lié avec son atome d'azote central au cycle triazine de la structure de formule (I), où
- Q¹ signifie H, un groupe hydroxyle, un groupe alkyle en C₁ à C₃₀ linéaire ou ramifié ou un substituent cyclique sous la forme d'un groupe cycloalkyle en C₅ à C₂₀, d'un groupe aryle en C₅ à C₂₀, d'un groupe aryle en C₅ à C₂₀ substitué par un groupe alkyle en C₁ à C₂₀, où dans chaque cas un ou plusieurs atomes de carbone peuvent être substitués par un ou plusieurs atomes d'oxygène, atomes de soufre, atomes d'azote substitués et/ou par un ou plusieurs groups du type -C(O)O-,-OC(O)-, -C(O)- et/ou -OC(O)O-, et/ou peuvent être fonctionnalisés par un ou plusieurs groupes hydroxyle et/ou groupes mercapto,
- R³, R⁴, R⁵ et R⁶ signifient, indépendamment les uns des autres, H, un groupe alkyle en C₁ à C₂₀ linéaire ou ramifié, un groupe cycloalkyle en C₅ à C₂₀, un groupe aryle en C₅ à C₂₀, un groupe aryle en C₅ à C₂₀ substitué par un groupe alkyle en C₁ à C₂₀, où dans chaque cas un ou plusieurs atomes de carbone peuvent être substitués par un ou plusieurs atomes d'oxygène, atomes de soufre et/ou atomes d'azote substitués et/ou par un ou plusieurs groups du type -C(O)O-, -OC(O)-, -C(O)- et/ou - OC(O)O-, et/ou peuvent être fonctionnalisés par un ou plusieurs groupes hydroxyle et/ou groupes mercapto ; ou un amide d'un acide carboxylique ou un imide d'un acide dicarboxylique cyclique, et
b) faire réagir ladite aminotriazine de formule générale (I) avec au moins une amine secondaire de formule générale (II)
(R⁷R⁸)NH
dans laquelle R⁷ et R⁸ sont un groupe alkyle en C₁ à C₂₀ linéaire ou ramifié, un groupe cycloalkyle en C₅ à C₂₀, un groupe phényle ou un groupe aryle en C₅ à C₂₀ substitué par un groupe alkyle en C₁ à C₂₀ ou des amines cycliques, ou dans laquelle R⁷ et R⁸ forment ensemble un cycle alkyle, où dans chaque cas un ou plusieurs atomes de carbone peuvent être substitués par un ou plusieurs atomes d'oxygène, atomes de soufre et/ou atomes d'azote substitués et/ou par un ou plusieurs groups du type -C(O)O-, -OC(O)-, -C(O)- et/ou -OC(O)O- et/ou peuvent être fonctionnalisés par un ou plusieurs groupes hydroxyle et/ou groupes mercapto, et dans laquelle R⁷ et R⁸ peuvent être identiques ou différents ;
c) la réaction ayant lieu en présence d'au moins un composé de formule générale (III)
[(R⁹R¹⁰)₂NH₂⁺]ₙXⁿ⁻
dans laquelle R⁹ et R¹⁰ sont un groupe alkyle en C₁ à C₂₀ linéaire ou ramifié, un groupe cycloalkyle en C₅ à C₂₀ ou un groupe aryle en C₅ à C₂₀ substitué par un groupe alkyle en C₁ à C₂₀ ou des amines cycliques, ou dans laquelle R⁹ et R¹⁰ forment ensemble un cycle alkyle, où dans chaque cas un ou plusieurs atomes de carbone peuvent être substitués par un ou plusieurs atomes d'oxygène, atomes de soufre et/ou atomes d'azote substitués et/ou par un ou plusieurs groups du type - C(O)O-, -OC(O)-, -C(O)- et/ou -OC(O)O- et/ou peuvent être fonctionnalisés par un ou plusieurs groupes hydroxyle et/ou groupes mercapto, et dans laquelle R⁹ et R¹⁰ peuvent être identiques ou différents,
R⁷, R⁸, R⁹ et R¹⁰ peuvent être identiques ou différents,
Xⁿ⁻ est un anion d'un acide organique ou inorganique, et
n ≥ 1, en particulier 1, 2, 3 ou 4.

2. Procédé selon la revendication 1, **caractérisé en ce que** l'au moins une amine secondaire du composé général (II) est utilisée en tant que liquide et/ou gaz et/ou gaz liquéfié.

3. Procédé selon l'une quelconque des revendications précédentes, **caractérisé en ce que** R⁷, R⁸ de l'au moins une amine secondaire de formule générale (II) sont choisis dans le groupe comprenant un groupe alkyle en C₁ à C₁₀ linéaire ou ramifié, un groupe cycloalkyle en C₅ à C₁₀ ou un groupe aryle en C₅ à C₁₀ substitué par un groupe alkyle en C₁ à C₁₀ ou **en ce que** R⁷, R⁸ forment ensemble un cycle alkyle en C₆ à C₁₀, où dans chaque cas un ou plusieurs atomes de carbone peuvent être substitués par un ou plusieurs atomes d'oxygène, atomes de soufre et/ou atomes d'azote substitués et/ou peuvent être fonctionnalisés par un ou plusieurs groupes hydroxyle et/ou groupes mercapto.

4. Procédé selon la revendication 3, **caractérisé en ce que** R⁷, R⁸ sont choisis dans le groupe comprenant les groupes méthyle, éthyle, n-propyle, isopropyle, n-butyle, s-butyle, t-butyle, isobutyle, pentyle, cyclopentyle, hexyle, cyclohexyle, 2-éthyl-hexyle, octyle, décyle, stéaryle, phényle, toloyle, xyloyle, hydroxyéthyle, hydroxypropyle, ou **en ce que** R⁷, R⁸ font partie d'un groupe morpholine.

5. Procédé selon l'une quelconque des revendications précédentes, **caractérisé en ce que** Xⁿ⁻ du composé de formule (III) est un anion choisi dans le groupe comprenant les halogénures, en particulier le chlorure, le bromure et le fluorure, le sulfate, le phosphate, le nitrate, le nitrite, le tétrafluoroborate, l'hexafluorophosphate, le polyfluoralcanesulfonate, le bis(trifluorométhylsulfonyl)-imide, le sulfate d'alkyle, l'alcanesulfonate, le sulfonate d'aryle, l'acétate.

6. Procédé selon l'une quelconque des revendications précédentes, **caractérisé en ce que** le rapport de l'au moins une triazine de formule générale (I) et d'une amine secondaire de formule générale (II) est au moins 1:5, de préférence au moins 1:10, de manière préférée entre toutes au moins 1:20.

7. Procédé selon l'une quelconque des revendications précédentes, **caractérisé en ce que** la quantité de l'au moins un composé de formule générale (III) est au moins 1% en moles, de préférence au moins 10 % en moles, plus préférablement au moins 50 % en moles, de manière préférée entre toutes au moins 100 % en moles, par rapport à l'au moins une triazine de formule générale (I).

8. Procédé selon l'une quelconque des revendications précédentes, **caractérisé en ce que** la réaction est réalisée à une température comprise dans la plage entre 120 et 280 °C, de préférence entre 150 et 230 °C, de manière préférée entre toutes entre 180 et 220 °C.

9. Procédé selon l'une quelconque des revendications précédentes, **caractérisé en ce que** la réaction est réalisée pendant au moins 1 h jusqu'à 24 heures.

10. Procédé selon l'une quelconque des revendications précédentes, **caractérisé en ce que** la triazine utilisée dans l'étape a) répond à la formule générale (Ia) 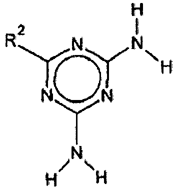 dans laquelle R² a la signification ci-dessus.

11. Procédé selon l'une quelconque des revendications précédentes, **caractérisé en ce que** la mélamine est utilisée en tant que triazine dans l'étape a).

12. Procédé selon l'une quelconque des revendications précédentes, **caractérisé en ce que** le composé obtenu répond à la formule générale (IV) 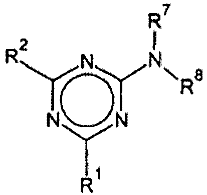 dans laquelle les substituants R¹, R² et R⁷, R⁸ ont les significations ci-dessus.

13. Procédé selon l'une quelconque des revendications précédentes, **caractérisé en ce que** le composé obtenu répond à la formule générale (IVa) 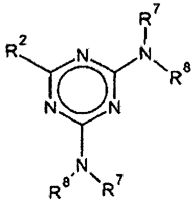 dans laquelle les substituants R² et R⁷, R⁸ ont les significations ci-dessus.

14. Procédé selon l'une quelconque des revendications précédentes, **caractérisé en ce que** le composé obtenu est choisi dans le groupe comprenant la N,N-dialkylaminotriazine et la N,N,N',N'-tétraalkylaminotriazine, en particulier la N,N-dialkylmélamine et la N,N,N',N'-tétralkylmélamine, la N,N-diméthylaminotriazine et la N,N,N',N'-tétraméthylaminotriazine, en particulier la N,N-diméthylmélamine et la N,N,N',N'-tétraméthylmélamine, la N,N-diéthylaminotriazine et la N,N,N',N'-tétraéthylaminotriazine, en particulier la N,N-diéthylmélamine et la N,N,N',N'-tétraéthylmélamine, la N-morpholinoaminotriazine, la N,N'-dimorpholinoaminotriazine, en particulier la N-morpholinomélamine, la N,N'-dimorpholinomélamine, la N,N-di-(hydroxyéthyl)-aminotriazine et la N,N,N',N'-tétra-(hydroxyéthyl)-aminotriazine, en particulier la N,N-di-(hydroxyéthyl)mélamine et la N,N,N',N'-tétra-(hydroxyéthyl)mélamine, la N-hydroxyéthyl-N-méthylaminotriazine, la N,N,N',N'-di-(hydroxyéthyl-méthyl)aminotriazine, la N-hydroxyéthyl-N-éthylaminotriazine, la N,N,N',N'-di-(hydroxyéthyl-éthyl)aminotriazine, en particulier la N-morpholinomélamine, la N,N'-dimorpholinomélamine, la N-hydroxyéthyl-N-méthylmélamine, la N,N,N',N'-di-(hydroxyéthyl-méthyl)mélamine, la N-hydroxyéthyl-N-éthylmélamine, la N,N,N',N'-di-(hydroxyéthyl-éthyl)mélamine.
